# EUROPÄISCHE PATENTANMELDUNG

(11) **EP 3 150 273 A1**
(43) Veröffentlichungstag der Anmeldung: **05.04.2017**
(21) Anmeldenummer: 16191642.4
(22) Anmeldetag: 30.09.2016
(51) Int. Cl.: B01D 69/08

(54) **FILTERMODUL EINER EXTRAKORPORALEN BLUTBEHANDLUNGSMASCHINE**

(30) Priorität: 02.10.2015 DE 102015116787
(71) Anmelder: B. Braun Avitum AG, 34212 Melsungen (DE)
(72) Erfinder: GOLDSTEIN, Manuel, 01139 Dresden (DE)
(74) Vertreter: Winter, Brandl, Fürniss, Hübner, Röss, Kaiser, Polte - Partnerschaft mbB

(57) **Zusammenfassung**

Die vorliegende Erfindung betrifft ein Filtermodul einer extrakorporalen Blutbehandlungsmaschine mit einem Filtergehäuse (2), in welchem ein Hohlfaserbündel (6) aufgenommen ist, dessen Hohlfasern (8) eine Hohlfaserwand (14) aus einer semipermeablen Membran aufweisen und wobei die Hohlfasern (8) in deren Längserstreckung eine Anzahl von Änderungen (10, 12) zumindest in deren Außendurchmesser aufzeigen. Erfindungsgemäß wird für den Außendurchmesser der jeweiligen Hohlfaser (8) eine Amplitude von 0,1 bis 1 mm ausgehend von deren Hohlfaserachse erreicht, wobei als Periodizität in Axialrichtung der Hohlfaser (8) ein Bereich von 1 bis 10 cm vorgesehen ist und an der Innenseite des Filtergehäuses (2) zumindest eine radiale Einschnürung (4) ausgebildet oder vorgesehen ist, durch welche das Faserbündel (6) lokal eingeschnürt wird.

## Beschreibung

Die vorliegende Erfindung betrifft ein Filtermodul einer extrakorporalen Blutbehandlungsmaschine, vorzugsweise Dialysemaschine mit einem Filtergehäuse, in welchem ein Hohlfaserbündel aufgenommen ist, dessen Hohlfasern eine Hohlfaserwand aus einer semipermeablen Membran aufweisen, wobei die Hohlfasern in deren Längserstreckung eine Anzahl von Änderungen zumindest in deren Außendurchmesser aufzeigen.

### Hintergrund der Erfindung

Ein entscheidendes Kriterium eines Filtermoduls (nachfolgend als Dialysator bezeichnet) insbesondere zur Verwendung in einer Blutreinigungsmaschine wie Dialysemaschine ist dessen Filterleistung. In sofern ist es ein Entwicklungsbestreben, diese Filterleistung zu steigern, ohne jedoch die Betriebssicherheit des Dialysators zu mindern oder dessen Handhabbarkeit zu verschlechtern. Eine Möglichkeit zur Steigerung der Filterleistung betrifft die Qualität der Filtermembran selbst, einschließlich deren Anordnung und Verteilung von Poren unterschiedlicher Porendurchmesser. Eine andere Möglichkeit liegt in der Verbesserung/Optimierung der Hohlfasergeometrie insbesondere in Faserlängsrichtung.

### Stand der Technik

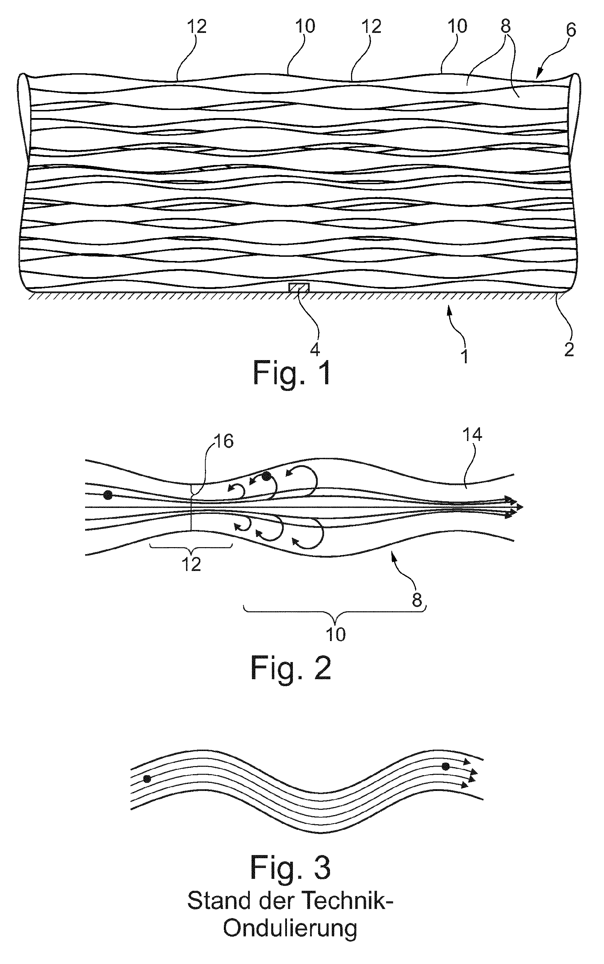

Aus dem Stand der Technik ist es grundsätzlich bekannt, Hohlfaserbündel innerhalb eines Dialysators in deren Gesamtquerschnitt abschnittsweise einzuschnüren, um so eine Art Strömungshindernis mit Drosselwirkung für eine Reinigungsflüssigkeit wie Dialysierflüssigkeit zu erzielen. Dadurch soll erreicht werden, dass eine Spül- bzw. Reinigungsflüssigkeit über den gesamten Filterquerschnitt strömt und nicht nur abschnittsweise Strömungspfade mit zufällig geringerem Strömungswiderstand nutzt. Des Weiteren ist es grundsätzlich bekannt, einzelne Hohlfasern, wie beispielhaft in der Fig. 3 dargestellt, innerhalb eines solchen Faserbündels in Wellenform auszubilden bzw. anzuordnen (Ondulierung). Ziel dieser bekannten Maßnahme ist es, durch eine geeignete Faserondulierung eine bessere filtratseitige (stromabwärtige) Umspülung der Hohlfasern zu erreichen. Außerdem sind im Faserbund in der Regel Abstandsfäden angeordnet, welche die Faserumspülung ebenfalls fördern.

Diese allgemein bekannten Maßnahmen haben jedoch Nachteile.

Die in der Regel 2-dimensionale Faserondulation birgt das Problem phasengleich aneinander liegender Fasern. Damit werden die Berührungsflächen nicht mehr oder ungenügend mit Spülflüssigkeit umspült. Der beabsichtigte Effekt wäre damit aufgehoben. Eine Aufhebung dieser insbesondere feed-seitigen (stromaufwärtigen) Laminate (d. h. das flächige Aneinander Haften/Kleben von Hohlfasermembranen) ist daher mit der bekannten Ondulierung nicht oder nur begrenzt möglich.

Untersuchungen dieser Problematik seitens der vorliegenden Anmelderin haben zu folgenden Erkenntnissen geführt:
Der transmembrane Stofftransport innerhalb eines vorzugsweise nach dem Gegenstromprinzip betriebenen, axial durchströmten Hohlfasermembran-Filtermoduls der einschlägigen Gattung (beispielsweise zur Hämodialyse) wird unter anderem dadurch vermindert, dass
   - filtratseitige Membranareale trotz Faserondulierung nicht maximal mit einer Spüllösung (z.B. Dialysierflüssigkeit) umströmt werden, da Fasern des Faserbündels örtlich über gewisse Faserlängsstrecken parallel und phasengleich ausgerichtet sind, sodass Wellenberge bzw. Wellentäler direkt in- und aneinander zu liegen kommen und
   - die feed-seitige Strömung trotz der genannten Faserondulierung im laminaren Strömungsbereich liegt, wodurch zu entfernende Substanzen (z.B. harnpflichtige Moleküle im Fall einer Dialyse) die Faserwandung nicht erreichen können, da bei zu geringer treibender Kraft (z.B. ein Konzentrationsgefälle) die verschiedenen Laminate nicht passiert werden können.

### Kurzbeschreibung der Erfindung

Ausgehend von dieser Problematik ist es die Aufgabe der vorliegenden Erfindung, die Geometrie einer Hohlfasermembran so zu verändern, dass dadurch eine Steigerung der Filterleistung eines damit ausgerüsteten Filtermoduls (Dialysators) erreicht werden kann.

Diese Aufgabe wird durch Filtermodul mit den Merkmalen des Anspruchs 1 gelöst. Vorteilhafte Ausgestaltungen der Erfindung sind Gegenstand der Unteransprüche.

Der Grundgedanke der vorliegenden Erfindung zur Lösung der vorstehenden Aufgabe besteht demnach darin, dafür Sorge zu tragen, dass
- inbesondere/zumindest filtrat-seitig (stromabwärtig) nur punktuelle/minimale Berührungspunkte zwischen den Hohlfasern bestehen, um so die Umströmung mit Spülflüssigkeit zu befördern (d.h. durch Verringerung des sogenannten Totraums (nicht umspültes Areal) erfolgt automatisch eine Verbesserung der Reinigungsleistung/Filtrierleistung des Filtermoduls) und
- insbesondere/zumindest feed-seitig (stromaufwärtig) eine (vor-) bestimmte/vertretbare Turbulenz/Durchmischung innerhalb der Strömung der zu reinigenden Flüssigkeit wie Blut innerhalb der Hohlfaser stattfindet, um die Wahrscheinlichkeit zu erhöhen, dass zu entfernende Substanzen radial nach außen, d.h. in Richtung Faserwandung der Hohlfaser transportiert werden (geringere Diffusionsstrecke bewirkt eine verbesserte Reinigungsleistung des Filters/Filtermoduls).

Im Konkreten wird der vorstehend formulierte Grundgedanke der vorliegenden Erfindung konstruktiv dadurch umgesetzt, in dem die Hohlfaser in deren Längserstreckung eine Anzahl von Änderungen/Variationen (weiter - enger) zumindest des Außendurchmessers erhält. Dadurch wird erreicht, dass zwei benachbarte Hohlfasern selbst in Parallellage nicht flächig (linienförmig) sondern im Wesentlichen punktuell aneinander anliegen.

Vorzugsweise erfolgen die Änderungen in Faserlängsrichtung periodisch, wodurch sich abwechselnd radiale Erweiterungen und radiale Einschnürungen längs der einzelnen Hohlfaser ergeben, die ggf. von äußeren Einschürungen auf ein Faserbündel, gebildet aus einer Vielzahl von erfindungsgemäßen Hohlfasern zusätzlich überlagert werden können.

Weiter vorzugsweise bleibt die Dicke der Hohlfaserwand in deren Längserstreckung (im Wesentlichen) konstant. Dies bedeutet, dass sich die konstruktive Gestaltung der Hohlfaser-Außendurchmesser auf die Hohlfaser-Innendurchmesser durchschlägt.

In anderen Worten ausgedrückt erhält die Hohlfasermembran in deren Längserstreckung eine Anzahl von Ausbauchungen und Einschnürungen bei (im Wesentlichen) konstanter Hohlfaser-Wandstärke. Dadurch wird erreicht, dass innerhalb der Hohlfaser in deren Längserstreckung eine entsprechende Anzahl an Innenquerschnittsverringerungen und nachfolgender Innenquerschnittserweiterungen mit entsprechender Düsenwirkung im Übergangsbereich gebildet wird. Eine, eine solche Hohlfaser durchströmende Flüssigkeit (z.B. Blut) erfährt im Bereich jeder dieser Innenquerschnittsverringerungen demzufolge eine Strömungsgeschwindigkeitszunahme mit nachfolgender Expansion im turbulenten Strömungsbereich. Dadurch können verbessert zu entfernende Substanzen innerhalb der die Hohlfaser durchströmenden Flüssigkeit an die innenseitige Faserwandung herangeführt werden.

### Figurenbeschreibung

Die Erfindung wird nachfolgend anhand eines bevorzugten Ausführungsbeispiels unter Bezugnahme auf die begleitenden Figuren näher erläutert.
Fig. 1 zeigt ein Filtermodul, vorzugsweise Dialysator, gemäß der Erfindung, der vorliegend zur Verwendung in einer Blutbehandlungsmaschine, vorzugsweise Dialysemaschine, angepasst ist,
Fig. 2 zeigt einen Längsabschnitt einer Hohlfaser gemäß einem bevorzugten Ausführungsbeispiel der vorliegenden Erfindung und
Fig. 3 zeigt einen Längsabschnitt einer Hohlfaser gemäß dem Stand der Technik.

In der Fig. 1 ist ein Filtermodul 1 schematisch dargestellt. Dieses hat folglich ein vorzugsweise zylinderförmiges Filtergehäuse 2, dessen axiale Enden mit jeweils einem Gehäusedeckel (nicht weiter gezeigt, weil aus dem Stand der Technik hinlänglich bekannt) verschlossen sind. Längs des Filtergehäuses 2 ist innenseitig eine Anzahl von axialbeabstandeten radialen Vorsprüngen 4 vorgesehen (nur ein Vorsprung ist beispielhaft dargestellt), durch welche der Innenquerschnitt des Filtergehäuses 2 lokal verkleinert wird.

In das Filtergehäuse 2 ist ein Bündel 6 aus Hohlfasern 8 eingesetzt, wodurch ein Gesamt-Strömungslumen innerhalb der Hohlfasern 8 und ein Gesamt-Strömungslumen außerhalb der Hohlfasern 8 innerhalb des Filtergehäuses 2 geschaffen werden. Beide Gesamt-Strömungslumen sind getrennt voneinander über Eingangs- und Ausgangsanschlüsse (nicht weiter gezeigt, weil aus dem Stand der Technik hinlänglich bekannt) im Filtergehäuse 2 und/oder in den Gehäusedeckeln mit einer Blutbehandlungsmaschine wie Dialysemaschine und an den Blutkreis eines Patienten anschließbar, derart, dass nach dem Gegenstromprinzip Blut durch die Hohlfasern 8 hindurch und Spülflüssigkeit durch das Filtergehäuse 2 außerhalb der Hohlfasern 8 hindurch geleitet werden kann.

Die radial nach innen vorragenden Vorsprünge 4 bewirken jeweils eine lokale Einschnürung des Hohlfaserbündels 6, wodurch in diesen eingeschnürten Bereichen der Gesamtströmungsquerschnitt außerhalb der Hohlfasern 8 verkleinert wird und dadurch eine Art Strömungshindernis mit höherem Strömungswiderstand geschaffen wird. Dies dient dazu, dass eine Spülflüssigkeit auf der axialen Einlassseite des Filtermoduls 1 das gesamte Strömungslumen außerhalb der Hohlfasern 8 möglichst gleichmäßig füllt.

Wie ferner aus der Fig. 1 zu ersehen ist, sind die Hohlfasern 8 nicht mit einem über deren Länge konstanten Außenquerschnitt ausgebildet, sondern weisen (periodisch) sich abwechselnde radiale Aufweitungen/Ausbauchungen 10 und Verengungen/Einschnürungen 12 zumindest am Hohlfaser-Außendurchmesser auf, die vorzugsweise kontinuierlich, ohne Ausbildung von Stufen oder Kanten ineinander übergehen. Die Hohlfasern 8 mit einem derartigen Außendurchmesserverlauf sind innerhalb des Hohlfaserbündels 6 bevorzugt so angeordnet, dass sich die Ausbauchungen 10 und Verengungen 12 unterschiedlicher Hohlfasern 8 in Wirrlage zueinander anordnen, derart, dass jeweils benachbarte Hohlfasern 8 im Wesentlichen nur punktuellen Kontakt zueinander in deren Längserstreckung erhalten.

Mit Bezug auf die Fig. 2 hat jede solche Hohlfaser 8 eine semidurchlässige Membranwand 14, die über deren Längserstreckung eine (im Wesentlichen) konstante Wandstärke/Wanddicke aufweist, sodass sich die radialen Aufweitungen 10 und Einengungen 12 des Hohlfaser-Außendurchmessers am Hohlfaser - Innendurchmesser widerspiegeln. Dadurch werden Hohlfaser-innenseitig düsenartige Verengungen 16 geschaffen, durch welche eine die Hohlfaser 8 durchströmende Flüssigkeit mit einer Geschwindigkeit strömt, die höher ist als im Bereich der radialen Ausbauchungen 10. Dies wiederum bewirkt insbesondere auf der stromabwärtigen Seite der radialen Einengungen 12 eine Verwirbelung der Flüssigkeitsströmung, natürlich bei hinreichender Kenntnis der Strömungsverhältnisse sowie der Viskosität der Flüssigkeit.

Durch die abwechselnd angeordneten Ausbauchungen 10 und Einengungen 12 bleiben benachbarte sowie aneinander anliegende Hohlfasern 8 im Wesentlichen auf Distanz und können so ausreichend von Spülflüssigkeit (Dialysierflüssigkeit) umströmt werden. Des Weiteren bewirkt diese besondere Konstruktion eine Strömung innerhalb der Hohlfaser 8 lokal im turbulenten Bereich, wodurch zu entfernende Stoffe besser an die semipermeable Membranwand 14 der Hohlfaser 8 gelangen. Beide Effekte tragen zu einer Steigerung der Filterleistung bei, ohne dass das Filtermodul 8 insgesamt vergrößert werden muss und dadurch unhandlicher werden würde.

Es ist bevorzugt, für den Außendurchmesser der Hohlfaser 8 eine Amplitude von 0,1 bis 1 mm vorzusehen. Als Periodizität in Axialrichtung der Hohlfaser 8 ist ein Bereich von 0,1 bis 10 cm vorteilhaft.

Zusammenfassend wird eine Hohlfaser offenbart mit einer Hohlfaserwand aus einer semipermeablen Membran vorzugsweise zur Verwendung in einem Filtermodul einer Blutreinigungsmaschine, wobei die Hohlfaser in deren Längserstreckung eine Anzahl von Änderungen zumindest in deren Außendurchmesser aufzeigt.

## Patentansprüche

1. Filtermodul einer extrakorporalen Blutbehandlungsmaschine, vorzugsweise Dialysemaschine, mit einem Filtergehäuse (2), in welchem ein Hohlfaserbündel (6) aufgenommen ist, dessen Hohlfasern (8) eine Hohlfaserwand (14) aus einer semipermeablen Membran aufweisen und wobei die Hohlfasern (8) in deren Längserstreckung eine Anzahl von Änderungen (10, 12) zumindest in deren Außendurchmesser aufzeigen, **dadurch gekennzeichnet, dass** für den Außendurchmesser der jeweiligen Hohlfaser (8) eine Amplitude von 0,1 bis 1 mm ausgehend von deren Hohlfaserachse erreicht wird, wobei als Periodizität in Axialrichtung der Hohlfaser (8) ein Bereich von 1 bis 10 cm vorgesehen ist und an der Innenseite des Filtergehäuses (2) zumindest eine radiale Einschnürung (4) ausgebildet oder vorgesehen ist, durch welche das Faserbündel (6) lokal eingeschnürt wird.

2. Filtermodul nach Anspruch 1, **dadurch gekennzeichnet, dass** die Änderungen (10, 12) in Hohlfaserlängsrichtung periodisch erfolgen, wodurch sich abwechselnd radiale Aufweiterungen (10) und radiale Einschnürungen (12) längs der einzelnen Hohlfaser (8) ergeben.

3. Filtermodul nach einem der vorstehenden Ansprüche, **dadurch gekennzeichnet, dass** die Stärke der Hohlfaserwand (14) in deren Längserstreckung im Wesentlichen konstant ist, wodurch sich der Innendurchmesser der Hohlfaser (8) in Abhängigkeit von den Änderungen des Außendurchmessers der Hohlfaser (8) im Wesentlichen gleichförmig mit ändert.

4. Filtermodul nach Anspruch 2 oder 3, **dadurch gekennzeichnet, dass** die Änderungen (10, 12) in Hohlfaser-Längsrichtung stufenlos, vorzugsweise abgerundet ausgeführt sind.

5. Filtermodul nach einem der Ansprüche 2 bis 4, **dadurch gekennzeichnet, dass** der Übergang von Einschnürungen (12) zu nachfolgenden Aufweitungen (10) so gestaltet ist, dass eine Fluidströmung innerhalb der Hohlfaser (8) mit bekannter Fluidviskosität und bei vorbestimmter Strömungsgeschwindigkeit verwirbelt wird.
